# EUROPEAN PATENT SPECIFICATION

(11) **EP 1 708 630 B1**
(45) Date of publication and mention of the grant of the patent: **11.11.2009**
(21) Application number: 05702478.8
(22) Date of filing: 21.01.2005
(51) Int. Cl.: A61B 17/70

(54) **VERTEBRAL OSTEOSYNTHESIS EQUIPMENT**
WIRBEL-OSTEOSYNTHESE-GERÄT
MATERIEL D'OSTEOSYNTHESE VERTEBRALE

(30) Priority: 27.01.2004 FR 0400745; 19.03.2004 US 554416 P; 22.10.2004 FR 0411266
(43) Date of publication of application: 11.10.2006
(73) Proprietor: Medicrea International, 01700 Neyron (FR)
(72) Inventor: SOURNAC, Denys, F-01600 REYRIEUX (FR); CAFFIERO, Jean-Philippe, F-69006 LYON (FR); CARLIER, François, F-44352 GUERANDE (FR)
(74) Representative: Jeannet, Olivier
(86) International application number: PCT/IB2005/000341
(87) International publication number: WO 2005/072632

(56) References cited:
- WO-A-00/15125
- WO-A-03/068088
- WO-A1-03/047441
- FR-A1- 2 697 742
- FR-A1- 2 827 498
- US-A- 4 946 458
- US-A- 5 882 350

## Description

The present invention concerns a vertebral osteosynthesis equipment.

A vertebral osteosynthesis equipment comprises generally bony anchoring members, such as pedicular screws, forceps or hooks, one or two linking rods, intended to be linked to these anchoring members, and connection parts of this or these linking rods to these anchoring members. The equipment may also contain crosslinks adjustable in length, which connect transversally two parallel linking rods to maintain these rods with respect to one another.

In a type of existing equipment, each anchoring element may include a proximal threaded stud whereon may be screwed a nut and each connection part may contain a rounded portion intended to surround a linking rod and two parallel wings drilled with holes. These wings are intended to be engaged on said proximal threaded stud and to be clamped, by means of this nut, against a bearing surface provided on the anchoring element, this clamping causing in turn the clamping of said rounded portion around the linking rod and thereby ensuring the longitudinal immobilisation of this rod with respect to the anchoring element.

In another type of existing equipment, each anchoring element may be "tulip-shaped", i.e. include a head wherein is provided a transversal passage for receiving a linking rod.

The anchoring members may be of "polyaxial" type, i.e. enable, before clamping, a joint of the connection part (proximal threaded stud or "tulip-shaped" head) with respect to the base portion of the anchoring element, intended to be inserted in the bone. This joint facilitates the assembly of the linking rods to the anchoring members.

In the existing "polyaxial" anchoring members, the joint is realised in the form of a ball joint with spherical portions, either by providing a sphere at the distal end of a threaded proximal stud and a cavity in said base portion, this cavity receiving the sphere of the stud and being closed at the proximal portion to retain this sphere, or by providing a sphere at the proximal end of the base portion and a cavity corresponding to the bottom of the "tulip-shaped" head, The documents US-A-4 946 458, WO 03/068088, US 5,882,350 or WO 00/15125 illustrate such materials.

The shortcoming of this type of joint lies in enabling only limited bottoming of the connection part, taking into account the stop of this part against the base portion at the edge closing the cavity receiving the sphere. This limited bottoming may compromise in certain cases the placement of a rod on anchoring members. Moreover, the contact surface of the sphere with said edge is restricted and the clamping of the rod generates relatively significant axial tension on this edge, which is not optimal from a mechanic viewpoint. Besides, the repeated stresses inflicted to the equipment once implanted, resulting from the movements of the patient, generate repeated frictions of the sphere against said edge, which leads to an undesirable risk of diffusion of metal particles in the organism.

Besides, the existing vertebral osteosynthesis equipment is intended to immobilise two vertebrae with respect to one another, to eliminate any relative movement of these vertebrae, or to restore the adequate position of a vertebra with respect to the other. For the realisation of this immobilisation, this equipment is designed for a perfectly rigid assembly of the linking rods with the anchoring members.

This rigid assembly may however not be desirable in all cases. It leads in particular to the application of significant stresses to anchoring bony zones of said anchoring members, as well as to increased stresses at vertebral joints situated of both sides of the vertebral segment treated, which may lead to degenerescences of these joints. Besides, this rigid assembly is not adapted to the treatment of non-degenerative affections, notably to the treatment of scoliosis in younger patients.

The document WO 03/047441 relates to a damping element which is not a polyaxial screw.

The purpose of the present invention is to remedy all these shortcomings.

Its main object is hence to provide a vertebral osteosynthesis equipment comprising at least one polyaxial anchoring element, wherein the connection part has a significant bottoming with respect to the base portion of the anchoring element intended to be attached to the bone, and wherein the risk of diffusion of metal particles in the organism is significantly reduced with respect to an existing equipment.

Another object of the invention is to provide a vertebral osteosynthesis equipment enabling non-rigid assembly possibly flexible assembly, of the linking rods with the anchoring members, with possible dampening effect of the movement of the mobile parts.

An additional object of the invention is to provide a vertebral osteosynthesis equipment enabling such non-rigid assembly, possibly flexible assembly, having a mobile portion whereof the localisation is close to the conditions of anatomic movements.

An additional object of the invention is to provide a vertebral osteosynthesis equipment having small space requirements in order to minimise the interventions necessary to realise the implantation.

The equipment concerned includes, in itself, bony anchoring members, such as pedicular screws, forceps or hooks, one or two linking rods, intended to be connected to these anchoring members, and connection assemblies of this or these rods to these anchoring members, at least one of these anchoring members being of the "polyaxial" type, i.e. including a connection assembly articulated with respect to a base portion intended to be attached to the bone.

According to the invention, said "polyaxial" anchoring element comprises an elongated rod-shaped junction portion connecting a portion of said connection assembly and said base portion, this junction portion having a flexible structure providing the joint of this connection assembly with respect to the base portion.

The joint of the connection assembly is thus not a ball joint with spherical portions, but provided by the single flexion of said junction portion. The bottoming of the connection assembly may be quite significantly greater than that of a connection assembly of an existing anchoring element. Moreover, the axial tension imparted between the connection assembly and the base portion after assembly applies to the junction portion globally and not to a restricted surface of a portion of a joint, as according to the previous technique.

The absence of contact of portions of joint on restricted surfaces enables to reduce quite significantly, possibly to eliminate, the risk of diffusion of metal particles in the organism, and of wear of the material. There results the possibility of allowing flexibility of said junction portion after implantation, enabling thus non-rigid assembly, possibly flexible assembly, of the linking rods with the anchoring members.

The junction portion enables several degrees of multidirectional movements of said connection assembly with respect to the base portion, in translation as well as in pivoting motion.

Besides, the joint according to the invention is significantly less cumbersome than a joint according to the previous technique, which enables not only to minimise the interventions to realise for the implantation but also to localise this joint in order to come as close as possible to the conditions of anatomic movements.

According to a possibility, the junction portion is formed by a part distinct from the part of said, connection assembly and of the base portion. The junction portion is then notably composed of a rod of flexible material.

According to another possibility, the junction portion is composed of an extension of the base portion or of said part of the connection assembly, made flexible by an appropriate shape and/or by slots or recesses. This extension has for instance a tubular structure and exhibit a helicoid slot, or exhibits stepped radial recesses, preferably offset angularly.

The junction portion and the links of said part of said connection assembly and of said base portion are designed so that said part of said connection assembly and said base portion are never in contact with one another.

This absence of contact eliminates the risk of diffusion of metal particles in the organism.

Alternately, the junction portion is slightly stretchable longitudinally and means are provided to stretch this junction portion slightly longitudinally, enabling to space the surfaces away from one another whereas said part of said connection assembly and the base portion contact one another.

These surfaces of said part and of the base portion are shaped to guide the movement of joint of this part with respect to the base portion. It may notably concern surfaces in portions of a sphere, respectively concave and convex, and provided coaxially to said junction portion.

These surfaces enable thus, when reducing vertebrae for osteosynthesis, to guide the movement of the part of said connection assembly with respect to the base portion in order not to subject the junction portion to excessive transversal stresses.

Along the same line, these same surfaces of the part of said connection assembly and of the base portion may be bordered by lateral bearing surfaces, enabling lateral wedging of said part with respect to the base portion.

Preferably, said anchoring element of "polyaxial" type comprises at least one part or a portion of part with elastically deformable structure, placed after assembly, between a part of the connection assembly and said base portion, this part or portion of part with elastically deformable structure enabling mobility of the connection assembly, and hence of the linking rod, with respect to the base portion, with a dampening effect.

Thus, in the equipment according to the invention, the connection assembly is not immobilised with respect to the base portion after assembly but may, relative to the elastic deformability of said part or portion of part, have a certain clearance with respect to the base portion in order to authorise limited movements of the vertebrae. The stresses imparted by the anchoring element on the anchoring bony zones are thus notably reduced, as well as the risks of excessive stresses at vertebral joints situated on both sides of the vertebral segment treated.

The invention will be better understood, and other characteristics and advantages thereof, will appear with reference to the appended schematic drawing, representing, for non-limiting exemplification purposes, several possible embodiments of parts contained in the equipment affected.
Figure 1 is an axial sectional view of the different elements forming an anchoring element and a connection assembly contained in this equipment, according to a first embodiment;
Figure 2 is a lateral view of this anchoring element, of this connection assembly and of a linking rod, after assembly;
Figure 3 is a sectional view of these anchoring element, connection assembly and linking rod along the line III-III of Figure 2;
Figure 4 is a top view of these anchoring element, connection assembly and linking rod;
Figures 5 to 7 are perspective exploded, side and sectional views, respectively of the anchoring element, of the connection assembly and of the linking rod according to a second embodiment, along the line VII - VII of Figure 6;
Figures 8 to 10 are similar views of the anchoring element, of the connection assembly and of the linking rod according to a third embodiment, and
Figures 11 and 12 are views of the anchoring element, of the connection assembly and of the linking rod according to a fourth embodiment, respectively in perspective before assembly and as a sectional view along the axis of the anchoring element.

By simplification, the portions or elements described for the first embodiment, which can be found identically or similarly in the other embodiments, will be designated by the same numeric references and will not described further.

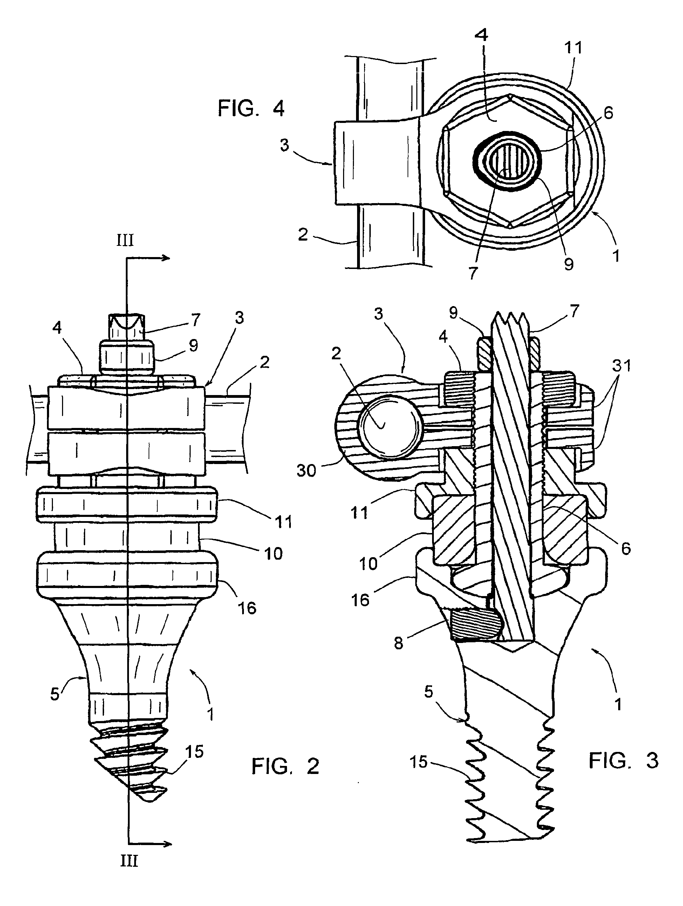

Figures 2 to 4 represent a polyaxial pedicular screw 1, a rod 2 for linking several of these screws 1, a yoke 3 for connecting this rod 2 to one of these screws 1 and a nut 4 enabling to assemble the linking rod 2 to this screw 1.

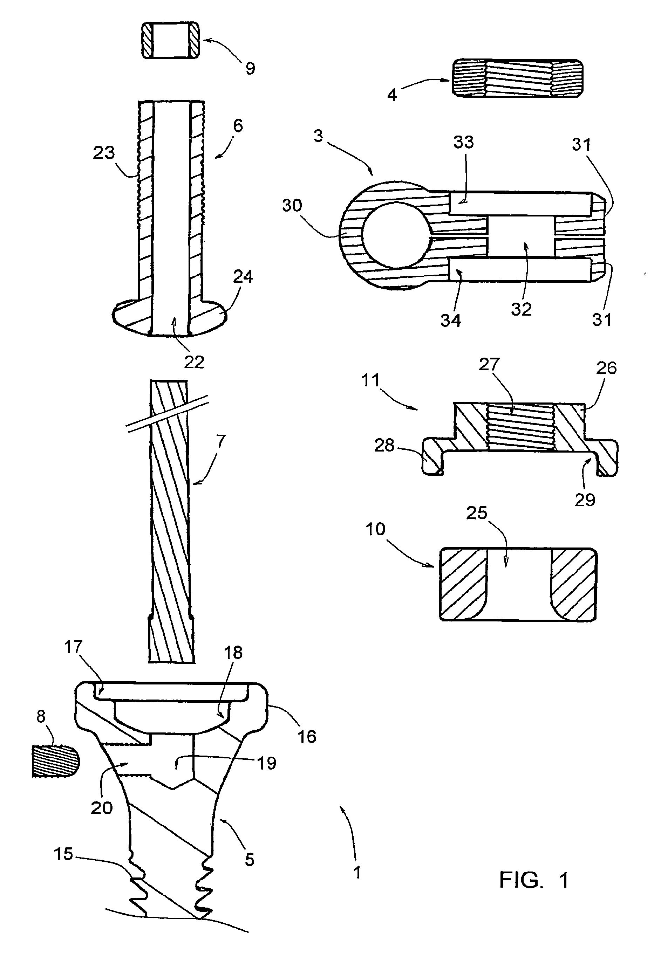

As shown more particularly on Figure 1, the screw 1 comprises a base part 5 in the form of a screw, a proximal threaded stud 6, an elongated element 7, for joining the base part 5 and the stud 6, a screwable plug 8 for assembling the element 7 to the base part 5 and a crimpable ring 9 for assembling the element 7 to the stud 6. The screw 1 comprises also a part 10 with elastically deformable structure and a ring 11 bearing against this part 10.

The stud 6, the yoke 3, the nut 4, the part 10 and the ring 11 form a connection assembly of a linking rod 2 to a base portion 5.

The base part 5 is metallic, notably made of titanium, as well as the stud 6, the rod 2, the yoke 3, the nut 4, the plug 8, the crimpable ring 9 and the ring bearing 11. It comprises a threaded cylindrical portion 15 enabling its insertion in the pedicle of a vertebra, and a widened head 16 intended to bear against this pedicle.

This head 16 comprises a cylindrical upper cavity 17, liable to receive the part 10 with a shrink-fit, a cylindrical intermediate cavity 18 with concave spherical bottom, liable to receive the base of the stud 6, and a lower bore 19, liable to receive an end of the element 7, these cavities 17, 18 and bore 19 being coaxial to the axis of the base portion 5.

The head 16 also comprises a tapered transversal perforation 20, emerging in the bore 19. As appears on Figure 3, the plug 8 is intended to be screwed in the perforation 20 in order to clamp the element 7 in the bore 19 and thus ensure the assembly of this element 7 with the base portion 5.

The stud 6 comprises an axial bore 22 liable to be traversed by the element 7, a proximal thread 23 and a wider base 24 with convex spherical distal face, whereas this base 24 may be received with a shrink-fit, but with possibility of movement, in the cavity 18.

The element 7 may notably be formed of braided polyester wires. After its engagement through the bore 22, this element 7 receives the ring 9, which is crimped thereon in order to maintain the base 24 in the cavity 18. The surplus portion of the element 7 is then cut beyond the ring 9.

The part 10 may notably be of silicon or of PMMA. It comprises an axial perforation 25 enabling its engagement on the stud 6, this bore 25 being flared at its lower portion to promote the bottoming of the stud 6 with respect to the base portion 5.

The bearing part 11 comprises an upper portion 26 drilled with a tapered bore 27 enabling to screw it on the stud 6 and a wider lower portion 28, delineating a lower cylindrical cavity 29 liable to receive the part 10 with a shrink-fit.

The part 10 is thus intended to be engaged on the stud 6 until it rests against the bottom of the cavity 17 and the part 11 is intended to be screwed on the stud 6 in order to clamp the part 10 therebetween and the base portion 5, independently of the clamping realised by the nut 4. The part 11 may be clamped in a controlled fashion, for instance by means of a torque wrench, according to the degree of dampening effect requested of the movement of the stud 5, relative to the characteristics of the patient (condition of the intervertebral disks, degree of vertebral instability, weight). The effect of this clamping is also to cause slight stretching of the element 7, which enables to move the convex face of the base 24 from the bottom concave of the cavity 18.

There results that the multidirectional movement of the stud 6 with respect to the base portion 5 is possible thanks to the sole flexibility of the element 7, according to a bottoming important, without any restricted bearing zone of the stud 6 against an edge of the base portion 5, and without any substantial friction of the adjacent surfaces of the stud 6 and of the base portion 5.

The linking rod 2 is cylindrical and exhibits such rigidity that it enables to maintain several vertebras relative to one another. This rod 2 is however deformable in order to be shaped relative to the vertebral correction to be carried out.

The yoke 3 comprises a rounded portion 30 intended to surround the linking rod 2 and two parallel lateral wings 31 drilled with holes 32 for the engagement of the yoke 3 on the stud 6. These wings 31 are mutually distant so that, in a spaced apart position, the rod 2 may be inserted and may slide in the portion 30, and, in a closing-in position provided by clamping the nut 4, they clamp the portion 30 around the rod 2, immobilising said rod with respect to the yoke 3.

As shown on Figure 1, each wing 31 exhibits a cavity 33, 34 on its outer face, intended for receiving the nut 4 and the bearing ring 11, respectively.

In practice, the number of screw 1 necessary to the treatment to realise is implemented in the pedicles of the vertebras affected, then the yokes 3, with the rod 2 engaged in the portions 30, are placed on the studs 6.

The significant bottoming of the studs 6 enables to facilitate quite notably this placement of the yokes 3 on its studs 6. During this operation, while the vertebras are reduced towards their osteosynthesis position, the heads 16 of the base portions 15 maintain transversally the bases 24 of the studs 6 and eliminate thus any risk of applying to the elements 7 excessive transversal stresses.

The nuts 4 are then clamped to bring the branches 31 together and thus clamp the rods 2 in the portions 30 of the yokes 3.

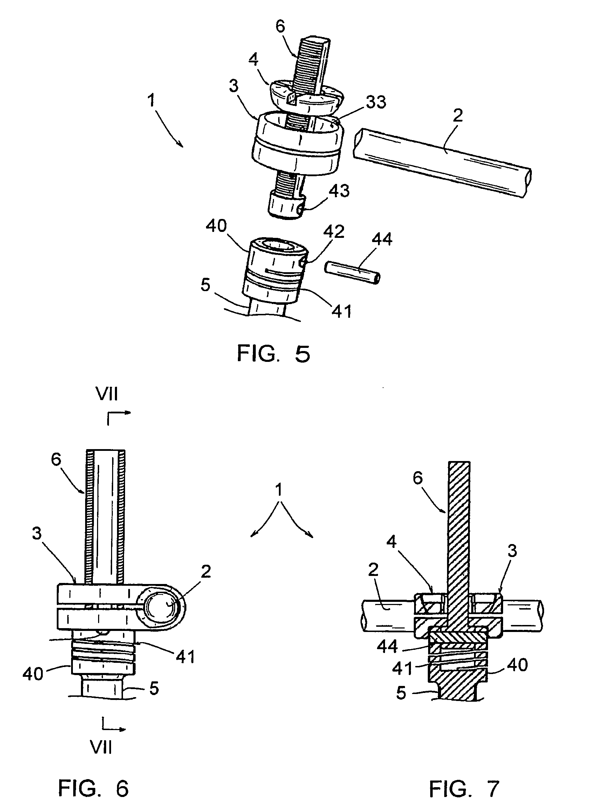

Figures 5 to 7 represent another embodiment of the osteosynthesis equipment according to the invention, also including a base part 5, a proximal threaded stud 6, a yoke 3 and a nut 4.

In such a case, the base part 5 comprises a proximal tubular extension 40 wherein is provided a helicoid slot 41.

This extension 40 is also drilled with two transversal coaxial holes 42 and the stud 6 comprises a transversal hole 43 facing, during the assembly of the stud 6 and of the base portion 5, these holes 42, whereas the holes 42, 43 may then receive a pin 44 pushed therethrough.

The slot 41 enables to confer the extension 40 the required flexibility to enable the stud 6 to bottom with respect to the base portion 5 and to enable a non-rigid assembly of a linking rod 2 to a base portion 5.

The yoke 3 and the nut 4 may be identical to those described previously or, as is represented, the nut 4 may exhibit an outer peripheral face in portion of a sphere and the yoke 3 may exhibit an upper cavity 33 of corresponding shape, wherein the nut 4 is totally engaged after complete screwing, as shown on Figure 7.

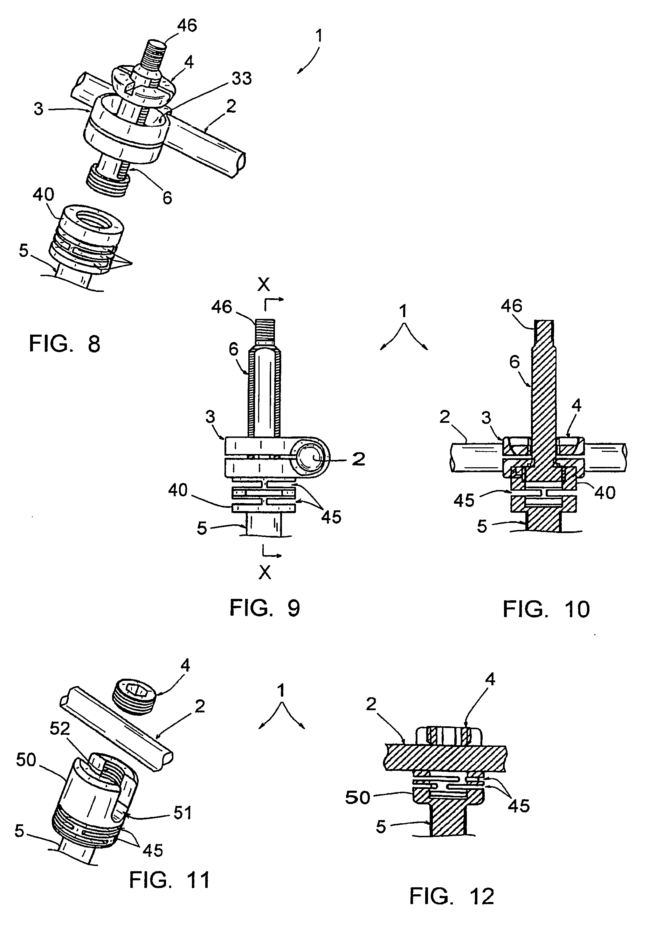

Figures 8 to 10 represent another embodiment of the osteosynthesis equipment according to the invention, also including a base portion 5 with proximal tubular extension 40, a proximal threaded stud 6, a yoke 3 and a nut 4.

In such a case, the proximal tubular extension 40 comprises stepped radial recesses 45, whereas each recess is provided on slightly less than half the circumference of the extension 40 and is provided with an angular offset with respect to the recesses 45 of the adjacent stages. These recesses 45 enable to delineate spaces authorising transversal deformation of the extension 40 in an "accordion"-like fashion, conferring this extension 40 the required flexibility.

The assembly of the stud 6 to the extension 40 may be realised by means of a pin as described previously, or, as represented, by screwing and gluing a threaded base of the stud 6 in the bore of the extension 40, which is also tapered.

The proximal threaded stud 6 may contain an upper stud 46 for screwing an extension (not represented) enabling to facilitate the descent of the yoke 3 - linking rod 2 assembly on the stud 6 when implanting the equipment.

Figures 11 and 12 represent a "tulip"-type screw 1, i.e. whereof the head 50 is widened and exhibits a transversal housing 51 for receiving a linking rod 2. This head 50 exhibits an axial tapered bore 52 provided in facing walls delineated by the transversal housing 51 and receives a nut 4 for locking the linking rod 2 in the housing 51.

In such a case, the base of the head 50 also comprises stepped radial recesses 45, whereas each recess is provided on slightly less than half the circumference of the head 50 and is provided with an angular offset with respect to the recesses 45 of the adjacent stages. These recesses 45 enable, as described previously, to delineate spaces authorising transversal deformation of the head 50 in an "accordion"-like fashion, conferring this head 50 the required flexibility.

As appears from the foregoing, the invention provides a vertebral osteosynthesis equipment wherein a part 6, 50 of the connection assembly has a significant bottoming with respect to the base portion 5 of the anchoring element 1 intended to be attached to the bone a nd wherein the risk of diffusion of metal particles in the organism is significantly reduced with respect to an existing equipment.

This vertebral osteosynthesis equipment enables moreover non-rigid assembly, possibly flexible assembly, of the linking rods 2 with the anchoring members 1, with possible dampening effect of the movement of the mobile parts.

Besides, this equipment has a mobile portion whereof the localisation is close to conditions of anatomic movements, and has small space requirements in order to enable to minimise the interventions necessary to realise the implantation.

It goes without saying that the invention is not limited to the embodiment described above for exemplification purposes but it extends to all the embodiments covered by the claims appended thereto.

In particular, the expression "connection part" should be understood in a wide meaning: it may be a proximal threaded stud 6 or a connection part in the form of a "tulip", i.e. including a housing for receiving a linking rod.

Similarly, the expression "base portion" 5 should be understood as any structure enabling the attachment of the anchoring element 1 with one vertebra, notably in the form of pedicular screw, forceps or hook.

## Claims

1. Vertebral osteosynthesis equipment comprising bony anchoring members, such as pedicular screws (1), forceps or hooks, one or two linking rods (2), intended to be connected to these anchoring members, and connection assemblies (6, 3, 4, 10, 11 ; 40, 50, 4) of this or these rods (2) to these anchoring members, at least one of these anchoring members (1) being of the "polyaxial" type, i.e. including a connection assembly (6, 3, 4, 10, 11 ; 40, 50, 4) articulated with respect to a base portion (5) intended to be attached to the vertebra ;
equipment **characterized in that** said "polyaxial" anchoring element (1) comprises a junction portion having an elongated rod-shape element (7) connecting a part (6) of said connection assembly (6, 3, 4, 10, 11) and said base portion (5), this junction portion having a flexible structure providing the joint of this connection assembly (6, 3, 4, 10, 11) with respect to the base portion (5).

2. Vertebral osteosynthesis equipment comprising bony anchoring members, such as pedicular screws (1), forceps or hooks, one or two linking rods (2), intended to be connected to these anchoring members, and connection assemblies (3, 4, 6 ; 50, 4) of this or these rods (2) to these anchoring members, at least one of these anchoring members (1) being of the "polyaxial" type, i.e. including a connection assembly (3, 4, 6 ; 50, 4) articulated with respect to a base portion (5) intended to be attached to the vertebra ;
equipment **characterized in that** said "polyaxial" anchoring element (1) comprises a junction portion formed by an extension (40) of the base portion (5) or by an extension of one part (50) of connection assembly (3, 4 ; 50, 4), connecting a part (6 ; 50) of said connection assembly (3, 4, 6 ; 50, 4) and said base portion (5), this junction portion having a flexible structure providing the joint of this connection assembly (3, 4, 6 ; 50, 4) with respect to the base portion (5).

3. Equipment according to claim 1, **characterized in that** the junction portion is formed by a part (7) distinct from the part (6) of said connection assembly (6, 3, 4, 10, 11) and of the base portion (5).

4. Equipment according to claim 3, **characterized in that** the junction portion is composed of a rod (7) of flexible material.

5. Equipment according to claim 2, **characterized in that** the extension (40) or the said one part (50) of connection assembly (3, 4, 6 ; 50, 4) has a tubular structure and exhibit a helicoid slot (41) or stepped radial recesses (45).

6. Equipment according to claim 1, **characterized in that** the junction portion and said part of said connection assembly and said base portion are designed so that said part of said connection assembly and said base portion are never in contact with one another.

7. Equipment according to claim 1, **characterized in that** the said part (6) of said connection assembly (6, 3, 4, 10, 11) and the said base portion (5) have respectives surfaces by which they contact one another, **in that** the junction portion (7) is slightly stretchable longitudinally, and **in that** means (6, 9, 3, 4, 10, 11) are provided to stretch this junction portion (7) slightly longitudinally, enabling to space said surfaces away from one another.

8. Equipment according to claim 7, **characterized in that** surfaces by which said part (6) of said connection assembly and the said base portion (5) contact one another, are shaped to guide the movement of joint of this connection part (6) with respect to the base portion (5).

9. Equipment according to claim 8, **characterized in that** said surfaces of the part (6) of said connection assembly and of the base portion (5) are bordered by lateral bearing surfaces, enabling lateral wedging of said part (6) with respect to the base portion (5).

10. Equipment according to any claims 1 to 9, **characterized in that** said anchoring element (1) of "polyaxial" type comprises at least one part or a portion of part (10) with elastically deformable structure, placed after assembly, between a part (11) of the connection assembly and said base portion (5), this part or portion of part (10) with elastically deformable structure enabling mobility of the connection assembly, and hence of the linking rod (2), with respect to the base portion (5), with a dampening effect.

## Patentansprüche

1. Material zur Wirbelosteosynthese, Knochenverankerungsorgane umfassend wie Pedikelschrauben (1), Klammern oder Haken, einen oder zwei Verbindungsstäbe (2), die dazu bestimmt sind, mit diesen Verankerungsorganen verbunden zu werden, und Gruppen zum Verbinden (6, 3, 4, 10, 11; 40, 50, 4) dieses Stabs oder dieser Stäbe (2) mit diesen Verankerungsorganen, wobei mindestens eines dieser Verankerungsorgane (1) vom Typ "polyaxial" ist, das heißt, eine in bezug auf einen Basisabschnitt (5) angelenkte Verbindungsgruppe (6, 3, 4, 11; 40, 50, 4) umfasst, der dazu bestimmt ist, am Wirbel befestigt zu werden;
wobei das Material **dadurch gekennzeichnet ist, dass** das "polyaxiale" Verankerungsorgan (1) einen Verbindungsabschnitt umfasst, der ein längliches Element in Stabform (7) aufweist, das ein Teil (6) der Verbindungsgruppe (6, 3, 4, 10, 11) und den Basisabschnitt (5) verbindet, wobei dieser Verbindungsabschnitt eine flexible Struktur hat, die das Anlenken dieser Verbindungsgruppe (6, 3, 4, 10, 11) in bezug auf den Basisabschnitt (5) erlaubt.

2. Material zur Wirbelosteosynthese, Knochenverankerungsorgane umfassend wie Pedikelschrauben (1), Klammern oder Haken, einen oder zwei Verbindungsstäbe (2), die dazu bestimmt sind, mit diesen Verankerungsorganen verbunden zu werden, und Gruppen zum Verbinden (3, 4, 6; 50, 4) dieses Stabs oder dieser Stäbe (2) mit diesen Verankerungsorganen, wobei mindestens eines dieser Verankerungsorgane (1) vom Typ "polyaxial" ist, das heißt, eine in bezug auf einen Basisabschnitt (5) angelenkte Verbindungsgruppe (3, 4, 6; 50, 4) umfasst, der dazu bestimmt ist, am Wirbel befestigt zu werden;
wobei das Material **dadurch gekennzeichnet ist, dass** das "polyaxiale" Verankerungsorgan (1) einen Verbindungsabschnitt umfasst, der von einer Verlängerung (40) des Basisabschnitts (5) oder von einer Verlängerung eines Teils (50) der Verbindungsgruppe (3, 4, 6; 50, 4) gebildet wird, der ein Teil (6; 50) der Verbindungsgruppe (3, 4, 6; 50, 4) und den Basisabschnitt (5) verbindet, wobei dieser Verbindungsabschnitt eine flexible Struktur hat, die das Anlenken dieser Verbindungsgruppe (3, 4, 6; 50, 4) in bezug auf den Basisabschnitt (5) erlaubt.

3. Material nach Anspruch 1, **dadurch gekennzeichnet, dass** der Verbindungsabschnitt von einem Teil (7) gebildet wird, das sich von dem Teil (6) der Verbindungsgruppe (6, 3, 4, 10, 11) und vom Basisabschnitt (5) unterscheidet.

4. Material nach Anspruch 3, **dadurch gekennzeichnet, dass** der Verbindungsabschnitt von einem Stab (7) aus einem flexiblen Werkstoff gebildet wird.

5. Material nach Anspruch 2, **dadurch gekennzeichnet, dass** die Verlängerung (40) oder das Teil (50) der Verbindungsgruppe (3, 4, 6; 50, 4) eine röhrenförmige Struktur hat und einen schraubenförmigen Schlitz (41) aufweist, oder stufenförmige radiale Aussparungen (45) aufweist.

6. Material nach Anspruch 1, **dadurch gekennzeichnet, dass** der Verbindungsabschnitt und das Teil der Verbindungsgruppe und der Basisabschnitt derart konstruiert sind, dass das Teil des Verbindungsabschnitts und der Basisabschnitt niemals miteinander im Kontakt sind.

7. Material nach Anspruch 1, **dadurch gekennzeichnet, dass** das Teil (6) der Verbindungsgruppe (6, 3, 4, 10, 11) und der Basisabschnitt (5) jeweilige Flächen aufweisen, durch die sie miteinander in Kontakt kommen, **dadurch**, dass der Verbindungsabschnitt (7) längs leicht streckbar ist, und **dadurch**, dass Mittel (6, 9, 3, 4, 10, 11) vorgesehen sind, um eine leichte längliche Streckung dieses Verbindungsabschnitts (7) durchzuführen, wobei diese Streckung eine Entfernung der Flächen voneinander erlaubt.

8. Material nach Anspruch 7, **dadurch gekennzeichnet, dass** die Flächen, durch die das Teil (6) der Verbindungsgruppe und der Basisabschnitt (5) miteinander in Kontakt kommen, ausgebildet sind, um die Gelenkbewegung des Verbindungsteils (6) in bezug auf den Basisabschnitt (5) zu führen.

9. Material nach Anspruch 8, **dadurch gekennzeichnet, dass** die Flächen des Teils (6) der Verbindungsgruppe und des Basisabschnitts (5) von seitlichen Abstützflächen gesäumt werden, die eine seitliche Verkeilung des Teils (6) in bezug auf den Basisabschnitt (5) erlauben.

10. Material nach einem der Ansprüche 1 bis 9, **dadurch gekennzeichnet, dass** das Verankerungsorgan (1) "polyaxialen" Typs mindestens ein Teil oder einen Teil-Abschnitt (10) mit elastisch deformierbarer Struktur umfasst, das bzw. der nach Montage zwischen einem Teil (11) der Verbindungsgruppe und dem Basisabschnitt (5) platziert ist, wobei dieses Teil oder dieser Teil-Abschnitt (10) mit elastisch deformierbarer Struktur eine Mobilität der Verbindungsgruppe und damit des Verbindungsstabs (2) in bezug auf den Basisabschnitt (5) mit Dämpfung erlaubt.

## Revendications

1. Matériel d'ostéosynthèse vertébrale, comprenant des organes d'ancrage osseux, tels que des vis pédiculaires (1), des pinces ou des crochets, une ou deux tiges de liaison (2) destinées à être reliées à ces organes d'ancrage, et des ensembles de connexion (6, 3, 4, 10, 11 ; 40, 50, 4) de cette ou ces tiges (2) à ces organes d'ancrage, au moins un de ces organes d'ancrage (1) étant du type "polyaxial", c'est-à-dire comprenant un ensemble de connexion (6, 3, 4, 10, 11 ; 40, 50, 4) articulé par rapport à une partie de base (5) destinée à être fixée à la vertèbre ;
matériel **caractérisé en ce que** ledit organe d'ancrage "polyaxial" (1) comprend une partie de jonction comportant un élément allongé en forme de tige (7) reliant une pièce (6) dudit ensemble de connexion (6, 3, 4, 10, 11) et ladite partie de base (5), cette partie de jonction ayant une structure flexible permettant l'articulation de cet ensemble de connexion (6, 3, 4, 10, 11) par rapport à la partie de base (5).

2. Matériel d'ostéosynthèse vertébrale, comprenant des organes d'ancrage osseux, tels que des vis pédiculaires (1), des pinces ou des crochets, une ou deux tiges de liaison (2) destinées à être reliées à ces organes d'ancrage, et des ensembles de connexion (3, 4, 6 ; 50, 4) de cette ou ces tiges (2) à ces organes d'ancrage, au moins un de ces organes d'ancrage (1) étant du type "polyaxial", c'est-à-dire comprenant un ensemble de connexion (3, 4, 6 ; 50, 4) articulé par rapport à une partie de base (5) destinée à être fixée à la vertèbre ;
matériel **caractérisé en ce que** ledit organe d'ancrage "polyaxial" (1) comprend une partie de jonction constituée par un prolongement (40) de la partie de base (5) ou par un prolongement d'une pièce (50) de l'ensemble de connexion (3, 4, 6 ; 50, 4) reliant une pièce (6 ;50) dudit ensemble de connexion (3, 4, 6 ; 50, 4) et ladite partie de base (5), cette partie de jonction ayant une structure flexible permettant l'articulation de cet ensemble de connexion (3, 4, 6 ; 50, 4) par rapport à la partie de base (5).

3. Matériel selon la revendication 1, **caractérisé en ce que** la partie de jonction est constituée par une pièce (7) distincte de la pièce (6) dudit ensemble de connexion (6, 3, 4, 10, 11) et de la partie de base (5).

4. Matériel selon la revendication 3, **caractérisé en ce que** la partie de jonction est constituée par une tige (7) de matériau flexible.

5. Matériel selon la revendication 2, **caractérisé en ce que** le prolongement (40) ou ladite pièce (50) de l'ensemble de connexion (3, 4, 6 ; 50, 4) a une structure tubulaire et présente une fente hélicoïdale (41), ou présente des évidements radiaux étagés (45).

6. Matériel selon la revendication 1, **caractérisé en ce que** la partie de jonction et ladite pièce dudit ensemble de connexion et ladite partie de base sont conçues de telle sorte que ladite pièce dudit ensemble de connexion et ladite partie de base ne sont jamais en contact l'une avec l'autre.

7. Matériel selon la revendication 1, **caractérisé en ce que** ladite pièce (6) dudit ensemble de connexion (6, 3, 4, 10, 11) et ladite partie de base (5) comportent des surfaces respectives par lesquelles elles viennent en contact l'une avec l'autre, **en ce que** la partie de jonction (7) est légèrement étirable longitudinalement, et **en ce que** des moyens (6, 9, 3, 4, 10, 11) sont prévus pour réaliser un léger étirement longitudinal de cette partie de jonction (7), cet étirement permettant un écartement desdites surfaces l'une par rapport à l'autre.

8. Matériel selon la revendication 7, **caractérisé en ce que** les surfaces par lesquelles ladite pièce (6) dudit ensemble de connexion et la partie de base (5) viennent en contact l'une avec l'autre sont conformées pour réaliser un guidage du mouvement d'articulation de la pièce de connexion (6) par rapport à la partie de base (5).

9. Matériel selon la revendication 8, **caractérisé en ce que** lesdites surfaces de la pièce (6) dudit ensemble de connexion et de la partie de base (5) sont bordées par des surfaces latérales d'appui, permettant un calage latéral de ladite pièce (6) par rapport à la partie de base (5).

10. Matériel selon l'une des revendications 1 à 9, **caractérisé en ce que** ledit organe d'ancrage (1) de type "polyaxial" comprend au moins une pièce ou une partie de pièce (10) à structure élastiquement déformable, placée, après montage, entre une pièce (11) de l'ensemble de connexion et ladite partie de base (5), cette pièce ou partie de pièce (10) à structure élastiquement déformable permettant une mobilité de l'ensemble de connexion, et donc de la tige de liaison (2), par rapport à la partie de base (5), avec amortissement.
